# EUROPEAN PATENT SPECIFICATION

(11) **EP 0 796 106 B1**
(45) Date of publication and mention of the grant of the patent: **19.03.2003**
(21) Application number: 95941606.6
(22) Date of filing: 21.12.1995
(51) Int. Cl.: A61K 38/26, C07K 14/605

(54) **PROTRACTED GLP-1 COMPOSITIONS**
GLP-1 ZUSAMMENSETZUNGEN MIT VERLÄNGERTER WIRKDAUER
COMPOSITIONS A BASE DE GLP-1 ET A ACTION PROLONGEE

(30) Priority: 23.12.1994 DK 147894
(43) Date of publication of application: 24.09.1997
(73) Proprietor: NOVO NORDISK A/S, 2880 Bagsvaerd (DK)
(72) Inventor: JENSEN, Ejvind, DK-3460 Birkerod (DK); JORGENSEN, Klavs, Holger, DK-2830 Virum (DK)
(74) Representative: Jorgensen, Dan
(86) International application number: DK9500516
(87) International publication number: WO96020005

(56) References cited:
- EP-A- 0 619 322
- WO-A-87/06941
- US-A- 5 180 522
- US-A- 5 214 035

## Description

### FIELD OF THIS INVENTION

The present invention relates to a composition containing GLP-1 compounds having protracted action and to a process for preparation thereof.

### BACKGROUND OF THIS INVENTION

Diabetes is characterized by an impaired glucose metabolism manifesting itself among other things by an elevated blood glucose level in the diabetic patients. Underlying defects lead to a classification of diabetes into two major groups, i.e., type I and type II diabetes. Type I diabetes, also designated insulin demanding diabetes mellitus (IDDM), arises when patients lack β-cells producing insulin in their pancreatic glands. Type II diabetes, also designated non-insulin dependent diabetes mellitus (NIDDM), occurs in patients with an impaired β-cell function besides a range of other abnormalities.

Type I diabetic patients are currently treated with insulin while the majority of type II diabetic patients are treated either with agents that stimulate β-cell function or with agents that enhance the tissue sensitivity of the patients towards insulin.

Glucagon-like peptide-1, also designated GLP-1, is a peptide sequence found as a constituent of mammalian proglucagon. In 1985, it was demonstrated that GLP-1(1-36) amide stimulates insulin release from isolated precultured rat pancreatic islets in the presence of glucose in a dose-dependent manner. This finding suggests that GLP-1(1-36) amide and related peptides might be useful in the treatment of type II diabetes. In recent years, particular interest has focused on GLP-1 fragments such as GLP-1(7-37) and GLP-1(7-36) amide and analogues and functional derivatives thereof. Hereinafter, these compounds are designated GLP-1 compounds.

It has been found that GLP-1 compounds such as GLP-1(7-37) and GLP-1(7-36) amide have a too fast action when administered to human subjects. Therefore, there is a need for compositions containing GLP-1 compounds and having a protracted action. The availability of such protracted compositions will spare the diabetics the chore and discomfort of multiple daily injections.

Apparently, some theoretical possibilities of controlling the duration of action of GLP-1(7-37) is described at the bottom of Column 6 in US patent specification No. 5,120,712. The possibilities mentioned therein are the use of polymers to complex or adsorb GLP-1(7-37), the selection of appropriate macromolecules (for example, protamine sulphate is mentioned among other), the incorporation of GLP-1(7-37) into particles of a polymeric material or the entrapment of GLP-1(7-37) in microcapsules.

A huge number of possible ways of preparing prolonged delivery of certain GLP-1 compounds is desribed in a European patent application having publication number 619,322. The possibilites mentioned therein are to add a polymer, to prepare an oil suspension, to add zinc (II), to add a metal, to add a basic polypeptide, to add a phenolic compound, to prepare an amorphous/crystalline formulation, or to use a liposome delivery system.

None of these known compositions are gels or thixotropic compositions.

One object of this invention is to provide compositions containing GLP-1 compounds and having a protracted action.

A further object of this invention is to provide compositions containing GLP-1 compounds and having a sufficient high stability, e.g. chemical stability and, especially, physical stability.

### BRIEF DESCRIPTION OF THIS INVENTION

Surprisingly, it has been found that compositions containing a GLP-1 compound and a phenolic and/or an alcoholic aromatic compound in certain concentrations result in a thixotropic gel showing a protracted release of the active GLP-1 compound.

### DETAILED DESCRIPTION OF THIS INVENTION

This invention deals with compounds having GLP-1 like activity herein referred to as GLP-1 compounds. GLP-1 compounds bind to the GLP-1 receptor (videProc.Nat. Acad.Sci.USA 89 (1992), 8641). Examples of specific GLP-1 compounds are polypeptides comprising the 7 - 34 amino acid sequence of GLP-1, viz. formula I: 
His-Ala-Glu-Gly-Thr-Phe-Thr-Ser-Asp-Val-Ser-Ser-Tyr-Leu-Glu-Gly-Gln-Ala-Ala-Lys-
Glu-Phe-Ile-Ala-Trp-Leu-Val-Lys
(I)
 or a peptide sequence derived from formula I without eliminating the GLP-1 like activity. The term GLP-1 compound also comprises derivatives of said polypeptides such as acid addition salts, carboxylate salts, lower alkyl esters, amides, lower alkyl amides and lower dialkyl amides.

The compositions of this invention are gels having thixotropic properties, said compositions containing a phenolic or an alcoholic aromatic compound. One way of preparing the thixotropic gels according to this invention is to mix the GLP-1 compound with a phenolic or an alcoholic aromatic compound in an aqueous medium. Preferably, the phenolic or alcoholic aromatic compound is a pharmaceutically acceptable antimicrobial preservative. Non-limiting examples of such compounds include benzyl alcohol, a cresol, e.g., m-cresol, a phenol, e.g., phenol or resorcinol, or a paraben, e.g., methyl paraben or propyl paraben. The compositions of this invention may contain both a phenolic or an alcoholic aromatic compound and a divalent metal ion, preferably in the form of a salt. A preferred ion is Zn(II). Other metal ions may also be used including Ca(II), Mg(II), Co(II), Mn(II), Fe(II), and Cu(II). For example, the divalent metal salts can be the chloride or another pharmaceutically acceptable salt. In one embodiment the composition contains 1 zinc ion per molecule of the GLP-1 compound or less and, preferably, they contain less than 0.4 zinc ion per molecule of the GLP-1 compound, more preferred they contain between 0.4 and 0.1 zinc ion per molecule of the GLP-1 compound and most preferred between 0.2 and above 0.1 zinc ion per molecule of the GLP-1 compound. Depending on which process has been used during the purification of the GLP-1 compound, it is, in some cases preferred to add an acetate and, in other cases, preferred to avoid the presence of acetate in the final GLP-1 composition.

The compositions of this invention can be prepared by using the GLP-1 compound in a concentration within a certain range. Consequently, a preferred embodiment of this invention is compositions containing not less than about 2 mg/ml, preferably not less than about 5 mg/ml, more preferred not less than about 10 mg/ml of a GLP-1 compound and, preferably, containing not more than about 100 mg/ml of a GLP-1 compound.

Another preferred embodiment of this invention relates to a thixotropic composition containing no compounds which are known to form thixotropic mixtures. It is novel that GLP-1 compounds and phenolic or alcoholic aromatic compounds which can safely be administered to human beings in medicaments can form thixotropic gels.

In addition to the specific ingredients which are to be present in the compositions of this invention, said composition may also, in addition to water, contain a pH buffering agent, an osmotic pressure controlling agent or other ancillary agents. In one embodiment the composition comprises glycerol.

The compositions of this invention can be used as an insulinotropic agent in the treatment of diabetes. The dosage to be administered to human subjects is conveniently determined by a physician. The dosage may be in the range 1 - 1,000 µg/kg/day. Normally, the compositions of this invention are administered subcutaneously or intramuscularly.

In one aspect the invention relates to the use of a GLP-1 compound for the manufacture of a medicament for the treatment of diabetes mellitus in a mammal in need of such treatment, said medicament comprising a composition as described above.

The features disclosed in the present description, examples and claims may, both separately and in any combination thereof, be material for realizing this invention in diverse forms thereof.

This invention is further illustrated by the following examples which are not to be construed as limiting, but merely as an illustration of some preferred features of this invention. Additional preferred embodiments of this invention are stated in the claims.

### Study of gelling and thixotropic properties

When evaluating compositions for their gelling and thixotropic properties, the following tests, which are performed at a temperature of 20-25°C, can be used:
Step 1:A cylindrical glass vial having a flat bottom, an inner diameter of about 6.4 mm and a height of about 4 cm is filled to a height of about 1 cm from the bottom with the composition which is to be tested. The filling can be made using a syringe. The glass vial used can be a 1 ml Clear Glass Vial With Caps from Waters, USA (part No. 78514).
Step 2: The vial is equipped with a cap and is stored for 24 hours.
Step 3:After removal of the cap, a glass ball is very cautiously placed at the top of the composition to be tested. This glass ball has a weight of about 17-18 mg and a diameter of about 2.4 mm. After standing for 1 hour, the glass ball should not sink more than about 5 mm.
Step 4 thereafter, the vial is placed in a vortex mixer (for example, a whirlimixer from Fisons Scientific Apparatus, England) and shaken. During this mixing step, the ball shall drop to the bottom.

For preferred compositions according to this invention the glass ball should not sink more than about 5 mm after standing for 5 hours in step 3 above, and more preferred it does not sink more than about 5 mm after standing for 24 hours in step 3 above.

A still further feature of preferred compositions according to this invention is that Step 4 is followed by the following steps:
Step 5: The vial is equipped with a cap and is stored for 24 hours.
Step 6: Thereafter, the vial is turned upside-down.
Step 7:Afler standing for 1 hour, the glass ball should not sink more than about 5 mm.

For preferred compositions according to this invention the glass ball should not sink more than about 5 mm after standing for 5 hours in step 7 above, and more preferred it does not sink more than about 5 mm after standing for 24 hours in step 7 above.

### Absorption studies.

The absorption of the GLP-1(7-37) compositions, described in the examples, were studied in pigs after subcutaneous injection. The compositions were made from a mixture of GLP-1(7-37) and a trace amount of ¹²⁵I-GLP-1(7-37). One composition was injected at one side of the neck and another composition at the other side in each of 6 pigs. The absorption was followed by external monitoring of the radioactivity remaining at the site of injection. The injections were performed by NovoPen® to a depth of 5 mm.

### Example 1

The zinc free gel composition of this invention designated A was: 20 mg/ml GLP-1(7-37), 16 mg/ml glycerol, and 3 mg/ml m-cresol (pH value: 7.2).

This composition was made by mixing 2.5 ml acidic GLP-1(7-37) solution (20 mg/ml) with 7.5 µl of m-cresol and 40 mg of glycerol, followed by adjustment of the pH value, which was made possible by the thixotropic properties of the gel that assumed low viscosity by stirring. A high viscosity gel was formed soon after stirring was stopped. 60 µl was injected in each pig.

### Example 2

The zinc containing gel composition of this invention designated B was: 20 mg/ml GLP-1(7-37), 0.5 mmol/l Zn⁺⁺, 16 mg/ml glycerol, and 3 mg/ml m-cresol. The molar ratio between Zn⁺⁺ and GLP-1(7-37) was 0.08.

This composition was made by mixing 1 ml of GLP-1(7-37) solution (40 mg/ml), adjusted to a pH value of 7.4, with 1 ml of a solution containing 6 g/l m-cresol, 32 g/l glycerol and 1 mmol/l zinc acetate. A high viscosity gel was formed soon after mixing. 80 µl were injected in each pig.

### Example 3

The zinc containing gel composition of this invention designated C was: 20 mg/ml GLP-1(7-37), 1 mmol/l Zn⁺⁺, 16 mg/ml glycerol, and 3 mg/ml m-cresol. The molar ratio between Zn ⁺⁺ and GLP-1(7-37) was 0.17.

This composition was made by mixing 1 ml of GLP-1(7-37) solution (40 mg/ml), adjusted to a pH value of 7.4, with 1 ml of a solution containing 6 g/l m-cresol, 32 g/l glycerol and 2 mmol/l zinc acetate. A high viscosity gel was formed soon after mixing. 80 µl were injected in each pig.

### Example 4

The zinc containing gel composition of this invention designated D was: 20 mg/ml GLP-1(7-37), 2 mmol/l Zn⁺⁺, 16 mg/ml glycerol, and 3 mg/ml m-cresol. The molar ratio between Zn ++ and GLP-1(7-37) was 0.33.

This composition was made by mixing 1 ml of GLP-1(7-37) solution (40 mg/ml), adjusted to a pH value of 8.7, with a 1 ml of a solution containing 6 mg/ml m-cresol, 32 g/l glycerol, and 4 mmol/l zinc acetate. A high viscosity gel was formed soon after mixing. 80 µl were injected in each pig.

### Example 5

As a non-gel, non-protracted solution of GLP-1(7-37) for use as a reference in the absorption studies, the following low concentrated zinc free GLP-1(7-37) composition designated REF was chosen: 1 mg/ml GLP-1(7-37), 16 mg/ml glycerol, 3 mg/ml phenol (pH value: 7.3).

The results of the absorption studies from Examples 1 - 5 are shown in the Table below.

**TABLE**

| Time after injection Hours | % Residual radioactivity | | | | |
|---|---|---|---|---|---|
| | Preparation A | Preparation B | Preparation C | Preparation D | Preparation REF |
| 0 | 100 | 100 | 100 | 100 | 100 |
| 1 | - | - | - | - | 42.6 |
| 1.5 | 70.6 | - | - | - | - |
| 2 | - | 64.1 | 76.6 | 94.7 | - |
| 3 | 36.8 | - | - | - | 4.5 |
| 4 | - | 44.4 | 67.0 | 91.3 | - |
| 5 | 10.3 | - | - | - | 1.9 |
| 6 | - | 32.7 | 60.8 | - | - |
| 6.5 | - | - | - | - | 1.7 |
| 7 | 3.4 | - | - | - | - |
| 15.5 | - | - | - | 59.2 | - |
| 21.5 | - | - | - | 40.3 | - |
| 24 | - | 2.4 | 12.8 | - | 1.1 |
| 40 | - | - | - | 9.1 | - |
| **T-50% (hours)*** | **2.3** | **3.3** | **8.4** | **19.3** | **0.8** |

| | | | | | |
|---|---|---|---|---|---|
| * Time until 50% of initial radioactivity remaining in the tissue, calculated on the basis of exponential disappearance between adjacent time points. | | | | | |

As appears from these data, the compositions of this invention are considerably more protracted than the reference solution.

## Claims

1. A composition containing a GLP-1 compound which composition contains a phenolic or an alcoholic aromatic compound, wherein GLP-1 and the phenolic or alcoholic aromatic compound are in concentrations to give a gel with thixotropic properties.

2. The composition according to claim 1, containing not less than about 5 mg/ml of a GLP-1 compound.

3. The composition according to any one of the claims 1-2, containing not less than 3 mg/ml of a phenolic or an alcoholic aromatic compound.

4. A composition containing a GLP-1 compound which composition is a gel with thixotropic properties containing not less than about 2 mg/ml of a GLP-1 compound and containing not less than 3 mg/ml of a phenolic or an alcoholic aromatic compound.

5. The composition according to any one of the claims 1-4, containing an osmotic pressure controlling agent.

6. The composition according to any one of claims 1-5, comprising glycerol.

7. The composition according to any one of the claims 1-6, containing not less than about 10 mg/ml of a GLP-1 compound.

8. The composition according to any one of the claims 1-7, containing not more than about 100 mg/ml of a GLP-1 compound

9. The composition according to any one of the claims 1-8, wherein the phenolic or alcoholic aromatic compound is independly selected from benzyl alcohol, a cresol, e.g., m-cresol, a phenol, e.g., phenol or resorcinol, or a paraben, e.g., methyl paraben or propyl paraben.

10. The composition according to claim 9, wherein the phenolic or alcoholic aromatic compound is m-cresol.

11. The composition according to any one of the claims 1-10, wherein the phenolic or alcoholic aromatic compound is a pharmaceutically acceptable antimicrobial preservative.

12. The composition according to any one of the claims 1-11, containing divalent metal ions, e.g. zinc, calcium, magnesium or cobalt ions.

13. The composition according to claim 12, wherein the metal ions are zinc ions.

14. The composition according to claim 13, containing 1 zinc ion per molecule of the GLP-1 compound or less and, preferably, they contain less than 0.4 zinc ion per molecule of the GLP-1 compound, more preferred they contain between 0.4 and 0.1 zinc ion per molecule of the GLP-1 compound and most preferred between 0.2 and above 0.1 zinc ion per molecule of the GLP-1 compound.

15. Use of a GLP-1 compound for the manufacture of a medicament for the treatment of diabetes mellitus in a mammal in need of such treatment, said medicament comprising a composition according to any of the preceding claims.

## Patentansprüche

1. Zusammensetzung enthaltend eine GLP 1-Verbindung, wobei die Zusammensetzung eine phenolische oder eine alkoholische aromatische Verbindung enthält, wobei GLP 1 und die phenolische oder alkoholische aromatische Verbindung in Konzentrationen vorliegen, um ein Gel mit thixotropischen Eigenschaften zu ergeben.

2. Zusammensetzung nach Anspruch 1, die nicht weniger als etwa 5 mg/ml einer GLP 1-Verbindung enthält.

3. Zusammensetzung nach einem der Ansprüche 1-2, die nicht weniger als 3 mg/ml einer phenolischen oder einer alkoholischen aromatischen Verbindung enthält.

4. Zusammensetzung enthaltend eine GLP 1-Verbindung, wobei die Zusammensetzung ein Gel mit thixotropischen Eigenschaften ist, die nicht weniger als etwa 2 mg/ml einer GLP 1-Verbindung und nicht weniger als 3 mg/ml einer phenolischen oder einer alkoholischen aromatischen Verbindung enthält.

5. Zusammensetzung nach einem der Ansprüche 1-4, die ein den osmotischen Druck kontrollierendes Mittel enthält.

6. Zusammensetzung nach einem der Ansprüche 1-5, umfassend Glycerol.

7. Zusammensetzung nach einem der Ansprüche 1-6, die nicht weniger als etwa 10 mg/ml einer GLP 1-Verbindung enthält.

8. Zusammensetzung nach einem der Ansprüche 1-7, die nicht mehr als etwa 100 mg/ml einer GLP 1-Verbindung enthält.

9. Zusammensetzung nach einem der Ansprüche 1-8, wobei die phenolische oder alkoholische aromatische Verbindung unabhängig voneinander ausgewählt ist aus Benzylalkohol, einem Cresol, z.B. m-Cresol, einem Phenol, z.B. Phenol oder Resorcinol, oder einem Paraben, z.B. Methylparaben oder Propylparaben.

10. Zusammensetzung nach Anspruch 9, wobei die phenolische oder alkoholische aromatische Verbindung m-Cresol ist.

11. Zusammensetzung nach einem der Ansprüche 1-10, wobei die phenolische oder alkoholische aromatische Verbindung ein pharmazeutisch geeignetes antimikrobielles Konservierungsmittel ist.

12. Zusammensetzung nach einem der Ansprüche 1-11, die divalente Metallionen enthält, z.B. Zink-, Calcium-, Magnesium- oder Kobaltionen.

13. Zusammensetzung nach Anspruch 12, wobei die Metallionen Zinkionen sind.

14. Zusammensetzung nach Anspruch 13, enthaltend 1 Zinkion pro Molekül der GLP 1-Verbindung oder weniger und vorzugsweise enthalten sie weniger als 0,4 Zinkionen pro Molekül der GLP 1-Verbindung, besonders bevorzugt enthalten sie zwischen 0,4 und 0,1 Zinkionen pro Molekül der GLP 1-Verbindung und am meisten bevorzugt zwischen 0,2 und oberhalb von 0,1 Zinkionen pro Molekül der GLP 1-Verbindung.

15. Verwendung einer GLP 1-Verbindung zur Herstellung eines Medikaments zur Behandlung von Diabetes mellitus in einem Säuger, der einer solchen Behandlung bedarf, wobei das Medikament eine Zusammensetzung entsprechend einem der vorhergehenden Ansprüche umfasst.

## Revendications

1. Une composition renfermant un composé GLP 1, laquelle composition comporte un composé aromatique alcoolique ou phénolique, dans laquelle GLP 1 et le composé aromatique alcoolique ou phénolique sont selon des concentrations pour donner un gel avec des propriétés thixotropes.

2. La composition selon la revendication 1, qui ne renferme pas moins d'environ 5 mg/ml d'un composé GLP 1.

3. La composition selon l'une quelconque des revendications 1 à 2, qui ne renferme pas moins de 3 mg/ml d'un composé aromatique alcoolique ou phénolique.

4. Une composition renfermant un composé GLP 1, laquelle composition est un gel avec des propriétés thixotropes qui ne contient pas moins d'environ 2 mg/ml d'un composé GLP 1 et qui ne contient pas moins de 3 mg/ml d'un composé aromatique alcoolique ou phénolique.

5. La composition selon l'une quelconque des revendications 1 à 4, renfermant un agent de régulation de la pression osmotique.

6. La composition selon l'une quelconque des revendications 1 à 5, qui renferme du glycérol.

7. La composition selon l'une quelconque des revendications 1 à 6, qui ne renferme pas moins d'environ 10 mg/ml d'un composé GLP 1.

8. La composition selon l'une quelconque des revendications 1 à 7, qui ne renferme pas plus d'environ 100 mg/ml d'un composé GLP 1.

9. La composition selon l'une quelconque des revendications 1 à 8, dans laquelle le composé aromatique alcoolique ou phénolique est choisi indépendamment parmi l'alcool benzylique, un crésol, par exemple le m-crésol, un phénol, par exemple le phénol ou le résorcinol ou un paraben, par exemple le méthyl-paraben ou le propyl paraben.

10. La composition selon la revendication 9, dans laquelle le composé aromatique alcoolique ou phénolique est le m-crésol.

11. La composition selon l'une quelconque des revendications 1 à 10, dans laquelle le composé aromatique alcoolique ou phénolique est un conservateur antimicrobien acceptable du point de vue pharmaceutique.

12. La composition selon l'une quelconque des revendications 1 à 11, renfermant des ions de métaux divalents, par exemple des ions de zinc, de calcium, de magnésium ou de cobalt.

13. La composition selon la revendication 12, dans laquelle les ions de métaux sont des ions de zinc.

14. La composition selon la revendication 13, renfermant 1 ion de zinc par molécule du composé GLP 1 ou moins et, de préférence, renfermant moins de 0,4 ion de zinc par molécule du composé GLP 1, plus particulièrement entre 0,4 et 0,1 ion de zinc par molécule du composé GLP 1 et, mieux encore, entre 0,2 et plus de 0,1 ion de zinc par molécule du composé GLP 1.

15. Utilisation d'un composé GLP 1 pour la fabrication d'un médicament pour le traitement du diabète mellitus chez un mammifère nécessitant un tel traitement, ledit médicament renfermant une composition selon l'une quelconque des revendications précédentes.
